Europäisches Patentamt

European Patent Office

Office européen des brevets

(19)

(11) **EP 0 639 370 B1**

(12) **EUROPÄISCHE PATENTSCHRIFT**

(45) Veröffentlichungstag und Bekanntmachung des Hinweises auf die Patenterteilung:
**15.10.1997 Patentblatt 1997/42**

(51) Int. Cl.$^6$: **A61K 7/40**

(21) Anmeldenummer: **94104918.1**

(22) Anmeldetag: **29.03.1994**

(54) **Verwendung einer gelförmigen Zubereitung als Hautschutzmittel sowie neue Hautschutzmittel**

Use of a gelified composition as skin protective agent and new skin protective agent

Utilisation d'une composition gélifiée comme agent protecteur de la peau et nouvel agent protecteur de la peau

(84) Benannte Vertragsstaaten:
**DE ES FR GB IT**

(30) Priorität: **19.06.1993 DE 4320401**

(43) Veröffentlichungstag der Anmeldung:
**22.02.1995 Patentblatt 1995/08**

(73) Patentinhaber: **Wella Aktiengesellschaft**
**64274 Darmstadt (DE)**

(72) Erfinder:
• **Hoch, Dieter**
**D-64319 Pfungstadt-Eich (DE)**
• **Gross, Paul**
**D-64295 Darmstadt (DE)**
• **Flemming, Ernst**
**D-63150 Heusenstamm (DE)**

(56) Entgegenhaltungen:
WO-A-94/08555         DE-A- 4 232 944
US-A- 4 956 170

• **RESEARCH DISCLOSURE, Nr.3343, November 1992 Seiten 880 - 887 M.W. HELIOFF 'PVM/MA Decadiene Crosspolymer: A New Thickener/Stabilizer'**

Bemerkungen:
Die Akte enthält technische Angaben, die nach dem Eingang der Anmeldung eingereicht wurden und die nicht in dieser Patentschrift enthalten sind.

## Beschreibung

Gegenstand der vorliegenden Erfindung ist die Verwendung einer gelförmigen Zubereitung zum Schutz und zur Pflege der Haut sowie neue Hautschutzmittel.

Die menschliche Haut, insbesondere die Haut der Hände, unterliegt durch den häufigen Kontakt mit seifen- oder tensidhaltigen Reinigungsmitteln einer starken Beanspruchung, die durch Quellvorgänge der Haut, verursacht durch den dauernden Kontakt mit Wasser, noch verstärkt wird. Hiervon sind insbesondere Hausfrauen sowie Personen mit handwerklicher Tätigkeit und im besonderen Maße auch Friseure betroffen, deren Hände häufig in Kontakt mit Dauerwellmitteln, Haarfärbepräparaten und tensidhaltigen Schäumen kommen. Durch Seifen und Tenside werden in der obersten Hautschicht, dem Stratum corneum, Schutzlipide ausgewaschen. Die Haut wird rauh und schuppig, trocknet aus und verliert ihre Elastizität. Eine derartige beanspruchte Haut ist in gesteigertem Maße anfällig für bakterielle Entzündungen und kumulativ toxische Ekzeme.

Um die Haut vor den vorstehend geschilderten Veränderungen zu schützen, wurden in der Vergangenheit Hautschutzcremes verwendet, welche üblicherweise als Wasser-in-Öl oder Öl-in-Wasser-Emulsionen vorlagen. Die emulsionsförmigen Hautschutzcremes haben den Nachteil, daß sie die Haut fettig und ölig machen, was besonders bei Hausfrauen, Friseuren, Ärzten und in vielen anderen Berufen als unangenehm und störend empfunden wird. So lassen sich zum Beispiel Gebrauchsgegenstände bei öliger und fettiger Haut der Hände schlecht handhaben.

Ein weiterer Nachteil von Hautschutzemulsionen besteht darin, daß sie durch Wasser leicht von der Haut wieder abgespült werden, was ein erneutes Eincremen erforderlich macht.

Die US-A-4 956 170 beschreibt ein Hautbehandlungsmittel mit einem hohen Alkoholgehalt, welches u.a. i) 0,4 - 2 Gew.% eines vernetzten Acrylsäurepolymeren, ii) ein Polydimethylsiloxan, iii) 0,05 bis 1 Gew.% eines nichtionischen Tensids und iv) 60-75 Gew.% Ethanol und/oder Isopropanol enthalten kann (Ansprüche 1 - 9 und Beispiele 1 - 2). Das Hautbehandlungsmittel soll antimikrobiell wirksam sein und trotz des hohen Alkoholgehaltes die Haut nicht austrocknen.

Es bestand daher die Aufgabe, eine Zubereitung zur Verwendung für den Schutz und die Pflege der Haut zur Verfügung zu stellen, welche die vorstehend genannten Nachteile bekannter Hautschutzcremes nicht aufweist. Die Zubereitung soll die Haut prophylaktisch schützen und sie gleichzeitig weich und geschmeidig pflegen.

Es wurde nun überraschenderweise gefunden, daß die Verwendung einer gelförmigen Zubereitung, welche dadurch gekennzeichnet ist, daß sie eine Kombination von

(A) 0,3 bis 4 Gewichtsprozent mindestens eines hochmolekularen Acrylsäurehomopolymers mit einem Molekulargewicht von 1.000.000 bis 4.000.000 und/oder eines mit Decadien vernetzten Methylvinylether-Maleinsäureanhydrid-Copolymers,

(B) 10 bis 50 Gewichtsprozent mindestens eines nicht-flüchtigen Polydimethylsiloxans,

(c) 0,1 bis 3 Gewichtsprozent mindestens eines nicht-ionischen und/oder anionischen Tensids und

(D) 10 bis 50 Gewichtsprozent Ethanol und/oder Isopropanol

enthält, zum Schutz und zur Pflege der Haut, die gestellte Aufgabe in hervorragender Weise erfüllt.

Gegenstand der vorliegenden Erfindung ist zudem ein gelförmiges Hautschutzmittel, welches dadurch gekennzeichnet ist, daß es eine Kombination von

(A) 0,3 bis 4 Gewichtsprozent mindestens eines hochmolekularen Acrylsäurehomopolymers mit einem Molekulargewicht von 1.000.000 bis 4.000.000 und/oder eines mit Decadien vernetzten Methylvinylether-Maleinsäureanhydrid-Copolymers,

(B) 10 bis 50 Gewichtsprozent mindestens eines nichtflüchtigen Polydimethylsiloxans,

(C) 0,1 bis 3 Gewichtsprozent mindestens eines nicht-ionischen und/oder anionischen Tensids und

(D) 40,01 bis 50 Gewichtsprozent Ethanol und/oder Isopropanol

enthält.

Sowohl die erfindungsgemäß verwendete Zubereitung als auch das erfindungsgemäße Hautschutzmittel gewährleisten den Schutz der Haut vor dem Austrocknen und vor Elastizitätsverlust. Die mit der erfindungsgemäß verwendeten Zubereitung oder dem erfindungsgemäßen Hautschutzmittel behandelte Haut ist weich und geschmeidig, fühlt sich jedoch nicht fettig oder ölig an.

Sowohl die verwendete Zubereitung als auch das erfindungsgemäße Hautschutzmittel enthalten die Komponente (A) bevorzugt in einer Menge von 0,4 bis 2 Gewichtsprozent. Ein geeignetes mit Decadien vernetztes Methylvinylether-Maleinsäureanhydrid-Copolymer, ist zum Beispiel das Handelsprodukt Stabileze® 06 der Firma ISP, Surrey, England.

Geeignete Acrylsäurehomopolymere sind beispielsweise solche mit einem Molekulargewicht von ca. 1.250.000 (z.B. das Handelsprodukt Acrisint® 410 der Firma Sigma, Bergamo, Italien), 3.000.000 (z.B. die Handelsprodukte Carbopol® 934, Carbopol® 2984 und Carbopol® 5984 der Firma B.F. Goodrich, Cleveland, USA oder Acrisint® 430 der Firma Sigma) oder 4.000.000 (z.B. die Handelsprodukte Carbopol® 940 der Firma B.F. Goodrich and Acrisint® 400 der Firma Sigma).

Zur Neutralisation der in der erfindungsgemäß verwendeten Zubereitung und dem erfindungsgemäßen Hautschutzmittel enthaltenen Polymere können beispielsweise verdünnte Lösungen von Natriumhydroxid, Ammoniak, Triethanolamin oder Diisopropanolamin verwendet werden.

Das erfindungsgemäße Hautschutzmittel und die verwendete Zubereitung weisen bevorzugt einen pH-Wert von 6,5 bis 7,5 auf.

Sowohl die erfindungsgemäß verwendete Zubereitung als auch das erfindungsgemäße Hautschutzmittel enthalten als Komponente (B) bevorzugt 20 bis 40 Gewichtsprozent mindestens eines nicht-flüchtigen Polydimethylsiloxans. Beispiele für geeignete nicht-flüchtige Polydimethylsiloxane sind Polydimethylsiloxane mit einer Viskosität von 50 bis 1.000 $mm^2 \cdot s^{-1}$, zum Beispiel die Handelsprodukte Abil® 500, Abil® 450 und Abil® 350 der Firma Goldschmidt, Essen, Deutschland, oder Silikon AK® 350, Silikon AK® 500 und Silikon AK® 1000 der Firma Wacker, München, Deutschland, sowie Dow Corning Fluid®-200, Dow Corning Fluid®-500 und Dow Corning Fluid®-350 der Firma Dow Corning, Midland, USA.

Die Zubereitung und das erfindungsgemäße Hautschutzmittel enthalten vorzugsweise 0,15 bis 0,5 Gewichtsprozent mindestens eines nicht-ionischen und/oder anionischen Tensids der Komponente (C). Das nicht-ionische Tensid ist bevorzugt ausgewählt aus oxethyliertem Rizinusöl, oxethyliertem hydrierten Rizinusöl oder Polyoxyethylensorbitanmonofettsäureester. Das, bevorzugt mit 30 bis 40 Mol Ethylenoxid, oxethylierte Rizinusöl kann beispielsweise in Form der Handelsprodukte Emulphor® EL-620 der Firma Rhone Poulenc, Paris, Frankreich, Emulphor® EL-719 (Rhone Poulenc) und Cremophor® EL der Firma BASF, Ludwigshafen, Deutschland verwendet werden. Das, bevorzugt mit 40 Mol Ethylenoxid oxethylierte, hydrierte Rizinusöl, kann beispielsweise in Form der Handelsprodukte Cremophor® RH 40 und Cremophor® RH 410 (BASF) verwendet werden. Als Polyoxyethylensorbitanmonofettsäureester, kann beispielsweise das mit 20 Mol Ethylenoxid oxethylierte Sorbitanmonopalmitat, zum Beispiel in Form des Handelsprodukts Tween® 40 der Firma ICI Americas Inc., Wilmington, USA, oder das mit 20 Mol Ethylenoxid oxethylierte Sorbitanmonostearat in Form des Handelsproduktes Tween® 60 (ICI Americas Inc.), verwendet werden. Als anionische Tenside werden bevorzugt Alkali-, Erdalkali-, Ammonium- oder Alkanolaminsalze von Alkansulfonaten, Alkylbenzolsulfonaten, Alkylethersulfaten, Alkylethercarboxylaten und Alkylsulfaten, wie beispielsweise $C_{12}$- bis $C_{18}$-Alkylsulfatnatriumsalze, insbesondere Natriumcetylstearylsulfat und Natriumlaurylsulfat, verwendet.

Die verwendete Zubereitung enthält die Komponente (D) bevorzugt in einer Menge von 40,01 bis 50 Gewichtsprozent.

Der Komponente (D) kommt bei der erfindungsgemäßen Verwendung der Zubereitung beziehungsweise bei der Anwendung des erfindungsgemäßen Hautschutzmittels eine besondere Bedeutung zu. Durch die lösungsvermittelnde Eigenschaft der Alkohole der Komponente (D) wird die Penetration der schützenden Komponente (B) in die obere Hautschicht besonders gefördert. Die Komponente (B) wird nach dem Verdunsten der Alkohole der Komponente (D) durch die Körperwärme in der oberen Hautschicht stabilisiert, wodurch die Schutzfunktion der erfindungsgemäß verwendeten Zubereitung und des erfindungsgemäßen Hautschutzmittels verstärkt wird. Die hautschützende Wirkung der erfindungsgemäß verwendeten Zubereitung ist daher besonders ausgeprägt, wenn die Komponente (D), wie in dem erfindungsgemäßen Hautschutzmittel, in einer Menge von 40,01 bis 50 Gewichtsprozent enthalten ist.

Sowohl die gemäß der vorliegenden Erfindung verwendete Zubereitung als auch das erfindungsgemäße Hautschutzmittel können zusätzlich alle diejenigen Bestandteile nthalten, die in Hautbehandlungsmitteln üblicherweise eingesetzt werden, insbesondere kationische, amphotere oder zwitterionische Tenside, beispielsweise Alkyltrimethylammoniumsalze, Alkylbetaine, Alkylaminobetaine, Alkylsulfobetaine und Fettsäurealkylamidobetaine in einer Menge von 0,01 bis bis 5,0 Gewichtsprozent; Sequestriermittel; Emulgatoren; Naturstoffe; Pigmente; Parfümöle in einer Menge von 0,5 bis 5,0 Gewichtsprozent; Trübungsmittel, wie zum Beispiel Ethylenglykoldistearat, in einer Menge von etwa 0,5 bis 5,0 Gewichtsprozent; Perlglanzmittel, wie zum Beispiel ein Gemisch aus Fettsäuremonoalkylolamid und Ethylenglykoldistearat, in einer Menge von etwa 1,0 bis 10,0 Gewichtsprozent; Verdickungsmittel, wie beispielsweise Kokosfettsäurediethanolamid oder Hydroxyalkylcellulose, in einer Menge von 0,5 bis 10,0 Gewichtsprozent; Puffersubstanzen, wie beispielsweise Natriumcitrat oder Natriumphosphat, in einer Menge von 0,1 bis 1,0 Gewichtsprozent; sowie Farbstoffe, wie zum Beispiel Fluorescein-Natriumsalz, Gelb ZN3 (C.I. 47 055), in einer Menge von 0,1 bis 1,0 Gewichtsprozent; weiterhin hautpflegende Zusätze, wie zum Beispiel Fettsäureester, Fettalkohole, Fettsäureglyceride; natürliche, modifizierte natürliche oder synthetische Polymere, wie zum Beispiel kationische, anionische oder nichtionische Cellulosederivate, Chitosan, kationische Chitin- oder Chitosanderivate, Polyvinylpyrrolidon oder Polymerisate von Acrylsäurederivaten; Pflegestoffe, wie zum Beispiel Lanolinderivate, Cholesterin Allantoin, Alpha-Bisabolol, Azulen und Pantothensäure, in einer Menge von 0,1 bis 10 Gewichtsprozent; sowie ferner Feuchthaltemittel; Lichtschutzmittel; Antioxidantien; Komplexbildner; kosmetische Öle und Wachse sowie Konservierungsstoffe, soweit solche Zusätze nützlich und zweckmäßig erscheinen und mit den Bestandteilen der verwendeten Zubereitung verträglich sind.

Die nachstehenden Beispiele sollen den Gegenstand der vorliegenden Erfindung näher erläutern.

**Beispiele**

**Beispiel 1: Hautschutzmittel in Gelform**

| | |
|---|---|
| 1,00 g | Acrylsäurehomopolymer mit einem Molekulargewicht von 3.000.000 |
| 15,00 g | Dimethylpolysiloxan mit einer Viskosität von 500 $mm^2 \cdot s^{-1}$ |
| 0,27 g | mit 40 Mol Ethylenoxid oxethyliertes hydriertes Rizinusöl |
| 28,41 g | Ethanol |
| 0,88 g | Ammoniak, 25 prozentige wäßrige Lösung |
| 54,44 g | Wasser, vollentsalzt |
| 100,00 g | |

Der pH-Wert wird mit wäßriger Ammoniaklösung auf 7 eingestellt.

**Beispiel 2: Hautschutzmittel in Gelform**

| | |
|---|---|
| 1,20 g | Acrylsäurehomopolymer mit einem Molekulargewicht von 3.000.000 |
| 20,00 g | Dimethylpolysiloxan mit einer Viskosität von 500 $mm^2 \cdot s^{-1}$ |
| 0,49 g | mit 20 Mol Ethylenoxid oxethyliertes Sorbitanmonopalmitat |
| 0,10 g | Allantoin |
| 23,68 g | Ethanol |
| 1,06 g | Ammoniak, 25 prozentige wäßrige Lösung |
| 53,47 g | Wasser, vollentsalzt |
| 100,00 g | |

Der pH-Wert wird mit wäßriger Ammoniaklösung auf 7 eingestellt.

**Beispiel 3: Hautschutzmittel in Gelform**

| | |
|---|---|
| 1,40 g | Acrylsäurehomopolymer mit einem Molekulargewicht von 4.000.000 |
| 18,00 g | Dimethylpolysiloxan mit einer Viskosität von 500 $mm^2 \cdot s^{-1}$ |
| 0,39 g | mit 20 Mol Ethylenoxid oxethyliertes Sorbitanmonopalmitat |
| 35,00 g | Isopropanol |
| 0,01 g | Azulen, 25 prozentige wäßrige Lösung |
| 1,23 g | Ammoniak, 25 prozentige wäßrige Lösung |
| 43,97 g | Wasser, vollentsalzt |
| 100,00 g | |

Der pH-Wert wird mit wäßriger Ammoniaklösung auf 7 eingestellt.

**Beispiel 4: Hautschutzmittel in Gelform**

| | |
|---|---|
| 0,400 g | Acrylsäurehomopolymer mit einem Molekulargewicht von 4.000.000 |
| 30,000 g | Dimethylpolysiloxan mit einer Viskosität von 1.000 $mm^2 \cdot s^{-1}$ |
| 0,450 g | mit 40 Mol Ethylenoxid oxethyliertes Rizinusöl |
| 45,000 g | Ethanol |
| 0,400 g | Parfümöl |
| 1,000 g | Glycerin |
| 0,001 g | Gelb ZN3 (C.I. 47 055) |
| 0,352 g | Ammoniak, 25 prozentige wäßrige Lösung |
| 22,397 g | Wasser, vollentsalzt |
| 100,000 g | |

Der pH-Wert wird mit wäßriger Ammoniaklösung auf 7 eingestellt.

**Beispiel 5: Hautschutzmittel in Gelform**

| | |
|---|---|
| 1,00 g | Acrylsäurehomopolymer mit einem Molekulargewicht von 3.000.000 |
| 15,00 g | Dimethylpolysiloxan mit einer Viskosität von 500 $mm^2 \cdot s^{-1}$ |
| 0,27 g | mit 40 Mol Ethylenoxid oxethyliertes hydriertes Rizinusöl |
| 45,00 g | Isopropanol |
| 0,10 g | Polyhexamethylen-biguanid-hydrochlorid, 20 prozentige wäßrige Lösung |
| 0,88 g | Ammoniak, 25 prozentige wäßrige Lösung |
| 37,75 g | Wasser, vollentsalzt |
| 100,00 g | |

Der pH-Wert wird mit wäßriger Ammoniaklösung auf 7 eingestellt.

**Beispiel 6: Hautschutzmittel in Gelform**

| | |
|---|---|
| 1,00 g | Acrylsäurehomopolymer mit einem Molekulargewicht von 3.000.000 |
| 15,00 g | Dimethylpolysiloxan mit einer Viskosität von 500 $mm^2 \cdot s^{-1}$ |
| 0,15 g | Natriumcetylstearylsulfat |
| 45,00 g | Ethanol |
| 0,10 g | alpha-Bisabolol |
| 0,88 g | Ammoniak, 25 prozentige wäßrige Lösung |
| 37,87 g | Wasser, vollentsalzt |
| 100,00 g | |

Der pH-Wert wird mit wäßriger Ammoniaklösung auf 7 eingestellt.

**Beispiel 7: Hautschutzmittel in Gelform**

| | |
|---|---|
| 1,40 g | Acrylsäurehomopolymer mit einem Molekulargewicht von 4.000.000 |
| 16,00 g | Dimethylpolysiloxan mit einer Viskosität von 500 $mm^2 \cdot s^{-1}$ |
| 0,50 g | Natriumlaurylpolyglykolethersulfat, 28 prozentige wäßrige Lösung |
| 23,68 g | Ethanol |

1,23 g     Ammoniak, 25 prozentige wäßrige Lösung

57,19 g    Wasser, vollentsalzt

100,00 g

Der pH-Wert wird mit wäßriger Ammoniaklösung auf 7 eingestellt.

Sämtliche in dieser Anmeldung angegebenen Prozentzahlen stellen Gewichtsprozente dar.

## Patentansprüche

1.  Verwendung einer gelförmigen Zubereitung mit einem Gehalt an einer Kombination von

    (A) 0,3 bis 4 Gewichtsprozent mindestens eines hochmolekularen Acrylsäurehomopolymers mit einem Molekulargewicht von 1.000.000 bis 4.000.000 und/oder eines mit Decadien vernetzten Methylvinylether-Maleinsäureanhydrid-Copolymers,

    (B) 10 bis 50 Gewichtsprozent mindestes eines nicht-flüchtigen Polydimethylsiloxans

    (C) 0,1 bis 3 Gewichtsprozent mindestens eines nicht-ionischen und/oder anionischen Tensids und

    (D) 10 bis 50 Gewichtsprozent Ethanol und/oder Isopropanol

    zum Schutz und zur Pflege der Haut.

2.  Verwendung nach Anspruch 1, dadurch gekennzeichnet, daß die verwendete Zubereitung 0,4 bis 2 Gewichtsprozent der Komponente (A) enthält.

3.  Verwendung nach einem der Ansprüche 1 oder 2, dadurch gekennzeichnet, daß die verwendete Zubereitung 20 bis 40 Gewichtsprozent der Komponente (B) enthält.

4.  Verwendung nach einem der Ansprüche 1 bis 3, dadurch gekennzeichnet, daß die verwendete Zubereitung 0,15 bis 0,5 Gewichtsprozent der Komponente (C) enthält.

5.  Verwendung nach einem der Ansprüche 1 bis 4, dadurch gekennzeichnet, daß die verwendete Zubereitung 40,01 bis 50 Gewichtsprozent der Komponente (D) enthält.

6.  Gelförmiges Hautschutzmittel, dadurch gekennzeichnet, daß es eine Kombination aus

    (A) 0,3 bis 4 Gewichtsprozent mindestens eines hochmolekularen Acrylsäurehomopolymers mit einem Molekulargewicht von 1.000.000 bis 4.000.000 und/oder eines mit Decadien vernetzten Methylvinylether-Maleinsäureanhydrid-Copolymers,

    (B) 10 bis 50 Gewichtsprozent mindestes eines nicht-flüchtigen Polydimethylsiloxans,

    (C) 0,1 bis 3 Gewichtsprozent mindestens eines nicht-ionischen und/oder anionischen Tensids und

    (D) 40,01 bis 50 Gewichtsprozent Ethanol und/oder Isopropanol

enthält.

7.  Mittel nach Anspruch 6, dadurch gekennzeichnet, daß es 0,4 bis 2 Gewichtsprozent der Komponente (A) enthält.

8.  Mittel nach einem der Ansprüche 6 oder 7, dadurch gekennzeichnet, daß es 20 bis 40 Gewichtsprozent der Komponente (B) enthält.

9.  Mittel nach einem der Ansprüche 6 bis 8, dadurch gekennzeichnet, daß es 0,15 bis 0,5 Gewichtsprozent der Komponente (C) enthält.

## Claims

1.  Use of a gel-form preparation containing a combination of

    (A) from 0.3 to 4% by weight of at least one high-molecular-weight acrylic acid homopolymer having a molecular weight of from 1,000,000 to 4,000,000 and/or of a methyl vinyl ether/maleic acid anhydride copolymer crosslinked with decadiene;
    (B) from 10 to 50% by weight of at least one non-volatile polydimethylsiloxane;
    (C) from 0.1 to 3% by weight of at least one non-ionic and/or anionic surfactant; and
    (D) from 10 to 50% by weight of ethanol and/or isopropanol

    for the protection and care of the skin.

2.  Use according to Claim 1, characterised in that the preparation used contains from 0.4 to 2% by weight of component (A).

3.  Use according to either Claim 1 or Claim 2, characterised in that the preparation used contains from 20 to 40% by weight of component (B).

4.  Use according to any one of Claims 1 to 3, characterised in that the preparation used contains from 0.15 to 0.5% by weight of component (C).

5. Use according to any one of Claims 1 to 4, characterised in that the preparation used contains from 40.01 to 50% by weight of component (D).

6. Gel-form skin-protecting agent, characterised in that it contains a combination of

(A) from 0.3 to 4% by weight of at least one high-molecular-weight acrylic acid homopolymer having a molecular weight of from 1,000,000 to 4,000,000 and/or of a methyl vinyl ether/maleic acid anhydride copolymer crosslinked with decadiene;
(B) from 10 to 50% by weight of at least one non-volatile polydimethylsiloxane;
(C) from 0.1 to 3% by weight of at least one non-ionic and/or anionic surfactant; and
(D) from 40.01 to 50% by weight of ethanol and/or isopropanol.

7. Agent according to Claim 6, characterised in that it contains from 0.4 to 2% by weight of component (A).

8. Agent according to either Claim 6 or Claim 7, characterised in that it contains from 20 to 40% by weight of component (B).

9. Agent according to any one of Claims 6 to 8, characterised in that it contains from 0.15 to 0.5% by weight of component (C).

## Revendications

1. Utilisation d'une préparation sous forme de gel présentant une teneur d'une combinaison de :

(A) 0,3 à 4 pour-cent en poids d'au moins un homopolymère d'acide acrylique à poids moléculaire élevé d'une valeur de 1.000.000 à 4.000.000 et/ou d'un copolymère méthylvinyléther-anhydride d'acide maléique réticulé avec du décadiène,

(B) 10 à 50 pour-cent en poids d'au moins un polydiméthylsiloxane non-volatile,

(C) 0,1 à 3 pour-cent en poids d'au moins un agent tensioactif non-ionique et/ou anionique, et

(D) 10 à 50 pour-cent en poids d'éthanol et/ou d'isopropanol,

pour assurer la protection et les soins de la peau.

2. Utilisation selon la revendication 1, caractérisée en ce que la préparation utilisée contient de 0,4 à 2 pour-cent en poids du composant (A).

3. Utilisation selon l'une des revendications 1 ou 2, caractérisée en ce que la préparation utilisée contient de 20 à 40 pour-cent en poids du composant (B).

4. Utilisation selon l'une des revendications 1 à 3, caractérisée en ce que la préparation utilisée contient de 0,15 à 0,5 pour-cent en poids du composant (C).

5. Utilisation selon l'une des revendications 1 à 4, caractérisée en ce que la préparation utilisée contient de 40,01 à 50 pour-cent en poids du composant (D).

6. Produit de protection des cheveux se présentant sous forme de gel, caractérisé en ce qu'il contient une combinaison de

(A) 0,3 à 4 pour-cent en poids d'au moins un homopolymère d'acide acrylique à poids moléculaire élevé d'une valeur de 1.000.000 à 4.000.000 et/ou d'un copolymère méthylvinyléther-anhydride d'acide maléique réticulé avec du décadiène,

(B) 10 à 50 pour-cent en poids d'au moins un polydiméthylsiloxane non-volatile,

(C) 0,1 à 3 pour-cent en poids d'au moins un agent tensioactif non-ionique et/ou anionique et

(D) 40,01 à 50 pour-cent en poids d'éthanol et/ou d'isopropanol.

7. Produit selon la revendication 6, caractérisé en ce qu'il contient de 0,4 à 2 pour-cent en poids du composant (A).

8. Produit selon l'une des revendications 6 ou 7, caractérisé en ce qu'il contient de 20 à 40 pour-cent en poids du composant (B).

9. Produit selon l'une des revendications 6 à 8, caractérisé en ce qu'il contient de 0,15 à 0,5 pour-cent en poids du composant (C).